# EUROPÄISCHE PATENTSCHRIFT

(11) **EP 1 073 469 B1**
(45) Veröffentlichungstag und Bekanntmachung des Hinweises auf die Patenterteilung: **17.11.2004**
(21) Anmeldenummer: 99916906.3
(22) Anmeldetag: 01.04.1999
(51) Int. Cl.: A61K 47/18, A61K 31/135

(54) **STABILE MITOXANTRON-LÖSUNGEN**
STABLE MITOXANTRON SOLUTIONS
SOLUTIONS STABLES DE MITOXANTRONE

(30) Priorität: 27.04.1998 DE 19818802
(43) Veröffentlichungstag der Anmeldung: 07.02.2001
(73) Patentinhaber: Baxter Healthcare S.A., 8304 Wallisellen (CH)
(72) Erfinder: MOSCHNER, Katrin, D-01129 Dresden (DE); WEINGART, Mario, D-01067 Dresden (DE); PIEROTH, Michael, D-01689 Weinböhla (DE); MORICK, Wolfgang, D-01259 Dresden (DE); WOLF-HEUSS, Elisabeth, D-74821 Mosbach (DE)
(74) Vertreter: Boos, Harald, Dr.
(86) Internationale Anmeldenummer: PCT/EP1999/002290
(87) Internationale Veröffentlichungsnummer: WO 1999/055375

(56) Entgegenhaltungen:
- EP-A- 0 236 822
- EP-A- 0 298 192
- WO-A-94/13274
- US-A- 3 248 291

## Beschreibung

### Technisches Gebiet

Die Erfindung betrifft Mitoxantron-Lösungen, die neben dem Wirkstoff Mitoxantron (1,4-Dihydroxy-5,8-bis[[2-[(2-hydroxyethyl)amino]ethyl]amino]anthrachinon-Hydrochlorid) einen Stabilisator enthalten und frei sind von Sulfitverbindungen, sowie die Verwendung dieser Lösungen zur Injektion und als Infusionslösungskonzentrat.

### Stand der Technik

Die Verbindung Mitoxantron ist ein Anthrachinon-Derivat mit der chemischen Bezeichnung 1,4-Dihydroxy-5,8-bis[[2-[(2-hydroxyethyl)amino]ethyl]amino]-anthrachinon-Hydrochlorid entsprechend der Formel 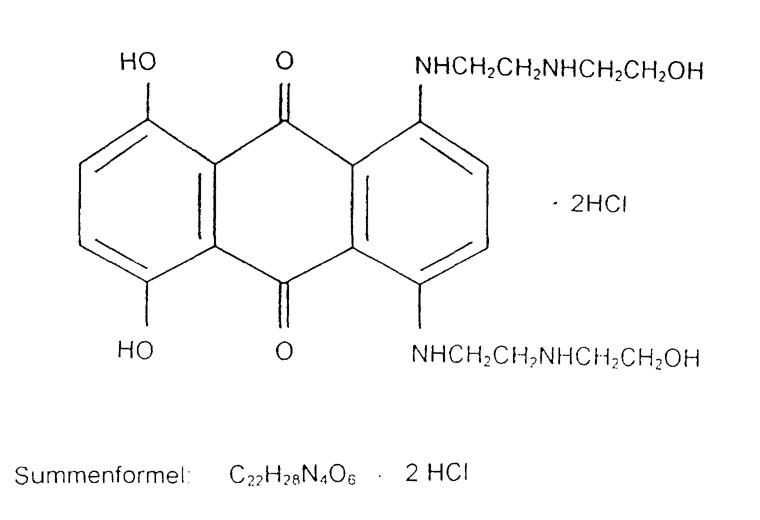 Mitoxantron zeigt antineoplastische, antivirale, antiprotozoale und immunmodulierende Eigenschaften. Die Verbindung ist als Zytostatikum von großem Nutzen, insbesondere in der Therapie von Mammakarzinom, malignen Lymphomen, akuten Leukämien, primärem Leberzellkarzinom und Ovarialkarzinom.

Als galenische Formulierung haben sich wäßrige Lösungen dieser Verbindung in der Anwendung durchgesetzt. Die Stabilität des Mitoxantrons in wäßriger Lösung ist jedoch begrenzt. Es ist bekannt, daß die Verbindung einer oxidativen Zersetzung unterliegt, sofern nicht Maßnahmen getroffen werden, diesen Abbau zu verlangsamen oder besser noch zu verhindern. Insbesondere Metallionen, selbst in geringsten Konzentrationen, verstärken diesen Verlauf.

Es hat sich gezeigt, daß diesem Abbau durch den Einsatz von Antioxidantien entgegengewirkt werden kann.

So schildert die EP-B-0 236 822 eine Zubereitung, die durch Kombination des Antioxidationsmittels Natriummetabisulfit, eines spezifischen pH-Wertes und der Chelatbildner Dinatriumedetat und Glycin stabilisiert wird.
Obwohl diese Zubereitung stabil ist, kann sie aber in Bezug auf Nebenwirkungen der Zusätze nicht befriedigen. Ein Nachteil der Rezeptur ist der Einsatz des im allgemeinen als Stabilisator sehr effektiven Natriummetabisulfits.
So sind Mitoxantron enthaltende Lösungen im Handel, für die ein Sulfitzusatz deklariert ist. Bei Anwendung von Präparaten, die Sulfit in den üblicherweise eingesetzten Konzentrationen enthalten, sind beim Menschen Überempfindlichkeitsreaktionen mit z. T. schwerwiegenden Nebenwirkungen berichtet worden. Für Asthmatiker mit Sulfitempfindlichkeit wurden z. B. lebensbedrohliche Komplikationen genannt.

Dazu kommt, daß die Verwendung einer metallionenbindenden Kombination aus wenigstens zwei Chelatbildnern zusätzliche Bestandteile der Zubereitung zuführt.

Nachteilig wirkt sich auch ein stark saurer pH-Bereich kleiner 3 aus, sowohl auf die Verträglichkeit in der Anwendung als auch auf die Stabilität (WANG, Da-Peng; LIANG, Gow-Zaw; TU, Yu-Hsing; Stability of mitoxantrone hydrochloride in solution, Drug Development and Industrial Pharmacy, 20(11), 1895-1903 (1994).

Die Erfindung bezweckt, die Nebenwirkungen zu vermeiden und eine unter normalen Lagerbedingungen und bei erhöhter Temperatur von 30°C über einen längeren Zeitraum stabile wäßrige Zubereitung zu erhalten, die frei ist von Sulfitverbindung.

### Darstellung der Erfindung

Im Gegensatz zum zitierten EP-B-0 236 822 wurde überaschenderweise gefunden, daß bereits Natriumedetat allein ausreicht, um Mitoxantron in wäßriger Lösung zu stabilisieren.
Die Aufgabe wird erfindungsgemäß gelöst mit einer wäßrigen Zubereitung, die besteht aus Mitoxantron-Hydrochlorid von 1 bis 5 mg/ml Lösung, einem Zusatz von 0,001-0,15 % Natriumedetat, Natriumchlorid, Natriumacetat und Essigsäure. Dabei wird ein pH-Wert im Bereich 3,0-4,5 erzielt.
Vorzugsweise wird das Natriumedetat in einer Menge von 0,04 % eingesetzt.

Die erfindungsgemäße pharmazeutische Zubereitung kann als Injektionslösung und Infusionslösungskonzentrat zur chemotherapeutischen Behandlung der genannten Krebskrankheiten verwendet werden. Sie vermeidet die genannten Nebenwirkungen und zeigt die für pharmazeutische Zubereitungen geforderte Stabilität.

### Wege zur Ausführung der Erfindung

Zur Herstellung der Lösung werden Natriumchlorid und Natriumacetat, Essigsäure, und Natriumedetat in sauerstoffarmes Wasser gegeben und gelöst. In einen Teil dieser Lösung wird das Mitoxantron-Hydrochlorid eingebracht und gelöst. Diese Wirkstofflösung wird der Lösung mit den Hilfsstoffen zugegeben und mit sauerstoffarmem Wasser auf das Endvolumen aufgefüllt.

Die so erhaltene Lösung wird filtriert und anschließend in Injektionsflaschen abgefüllt, verschlossen und verbördelt.

Zur Sterilisation benutzt man übliche, keimdichte Filter, wie beispielsweise Membranfilter mit einer Porengröße von 0,2 µm. Die verwendeten Gefäße werden zuvor in üblicher Weise sterilisiert. Das eingesetzte Wasser muß steril und pyrogenfrei und den Anforderungen des Europäischen Arzneibuches 1997 entsprechen. Die Injektionsgefäße bestehen zweckmäßig aus Glas der Glasart I gemäß den Anforderungen des Europäischen Arzneibuches 1997.
Die Lösungsmengen in den jeweiligen Gefäßen liegen pro Gefäß zwischen 2,5 ml und 50 ml, vorzugsweise zwischen 5 ml und 15 ml. Die Mitoxantron-Menge pro Gefäß beträgt 5 bis 100 mg, vorzugsweise 10 bis 30 mg.
Bei Abfüllung der Injektionslösung unter aseptischen Bedingungen werden die Flaschen zur Entfernung des Sauerstoffs ausreichend begast, mit Injektionsstopfen verschlossen und verbördelt.

Die nachfolgenden Beispiele sollen die Erfindung näher erläutern.

### Beispiel 1

### Injektionsflasche mit 5 ml einer 0,2 %igen Mitoxantron-Injektionslösung

### Zusammensetzung

1 ml Injektionslösung enthält:

| | |
|---|---|
| Mitoxantron-Hydrochlorid | 2,328 mg |
| Natriumchlorid | 8,000 mg |
| Natriumacetat×3 H₂O | 0,085 mg |
| Dinatriumedetat | 0,040 mg |
| 1N-Essigsäure | 7,680 µl |
| Wasser für Injektionszwecke zu | 1 ml |

### Herstellung der Lösung

Zur Herstellung der Injektionslösung werden Natriumchlorid, Natriumacetat, Essigsäure und Dinatriumedetat in Wasser gegeben und gelöst. Der Sauerstoffgehalt des Wassers wurde zuvor in geeigneter Weise verringert. Die Lösung wird geteilt und in einem Teil Mitoxantron-Hydrochlorid gelöst. Die das Mitoxantron enthaltende Lösung wird mit der verbliebenen Lösung zusammengegeben und mit sauerstoffarmem Wasser auf das Endvolumen aufgefüllt.
Eine Sterilisation der Lösung durch Filtration über Membranfilter schließt sich an.

### Beispiel 2

### Injektionsflasche mit 12,5 ml einer 0,2 %igen Mitoxantron-Lösung

Die Zusammensetzung und Herstellung der Mitoxantron-Lösung entspricht der gemäß Beispiel 1.

| Stabilitätsprüfergebnisse | | | | | |
|---|---|---|---|---|---|
| Rezeptur | Lagertemp. | Summe der Verunreinigungen in % | | | |
| | in °C | Ausgangswert | nach 6 Monaten | nach 18 Monaten | nach 24 Monaten |
| Beispiel 2 | 25 | 0,60 | 0,75 | 0,72 | 1,39 |
| wie Bsp.2, ohne Natriumedetat | 25 | 0,52 | 0,57 | 1,73 | 2,3 |
| Beispiel 2 | 30 | 0,60 | 0,82 | 1,78 | 2,00 |
| wie Bsp.2, ohne Natriumedetat | 30 | 0,52 | 0,93 | 2,74 | 4,16 |
| Beispiel 2 | 40 | 0,60 | 1,45 | - | - |
| wie Bsp.2, ohne Natriumedetat | 40 | 0,52 | 2,72 | - | - |

## Patentansprüche

1. Stabile Mitoxantron-Lösungen mit 1 bis 5 mg/ml Lösung an Mitoxantron-Hydrochlorid, 0,001 bis 0,15 % Natriumedetat und als weitere Bestandteile Natriumchlorid, Natriumacetat und Essigsäure enthaltend.

2. Stabile Mitoxantron-Lösungen nach Anspruch 1, **dadurch gekennzeichnet, daß** der Gehalt an Natriumedetat vorzugsweise 0,04 % beträgt.

3. Stabile Mitoxantron-Lösungen nach Anspruch 1, **dadurch gekennzeichnet, daß** der Gehalt an Dinatriumedetat vorzugsweise 0,004 % beträgt.

4. Verfahren zur Herstellung stabiler Mitoxantron-Lösungen gemäß den Ansprüchen 1 bis 3, **dadurch gekennzeichnet, daß** Natriumchlorid, Natriumacetat, Essigsäure und Natriumedetat in sauerstoffarmem Wasser gelöst werden, in einen Teil der Lösung Mitoxantron-Hydrochlorid eingebracht wird, die Lösungen vereinigt und auf das Endvolumen mit einem Gehalt von 1 bis 5 mg Mitoxantron-Hydrochlorid/ml Lösung und 0,001 bis 0,15 % Natriumedetat/ml Lösung eingestellt werden.

5. Verfahren nach Anspruch 4, **dadurch gekennzeichnet, daß** vorzugsweise 0,04 % Natriumedetat zugesetzt wird.

6. Verfahren nach Anspruch 4, **dadurch gekennzeichnet, daß** vorzugsweise 0,004 % Dinatriumedetat zugesetzt wird.

7. Verwendung der stabilen Mitoxantron-Lösungen gemäß einem der Ansprüche 1 bis 3 zur Herstellung eines Arzneimittels zur Anwendung bei Krebserkrankungen.

8. Arzneimittel zur Anwendung bei Krebserkrankungen, **dadurch gekennzeichnet, daß** es 1 bis 5 mg Mitoxantron-Hydrochlorid/ml Lösung, Natriumedetat in einer Menge von 0,001 bis 0,15 %, bezogen auf Mitoxantron-Hydrochlorid-Lösung, und als weitere Bestandteile Natriumchlorid, Natriumacetat und Essigsäure enthält.

9. Arzneimittel gemäß Anspruch 8, **dadurch gekennzeichnet, daß** es vorzugsweise 0,04 % Natriumedetat enthält.

10. Arzneimittel gemäß Anspruch 8, **dadurch gekennzeichnet, daß** es vorzugsweise 0,004 % Dinatriumedetat enthält.

## Claims

1. Stable mitoxantrone solutions containing a 1 to 5 mg/ml solution of mitoxantrone hydrochloride, 0.001 to 0.15% of sodium edetate and, as further constituents, comprising sodium chloride, sodium acetate and acetic acid.

2. Stable mitoxantrone injection solutions according to Claim 1, **characterized in that** the content of sodium edetate is preferably 0.04%.

3. Stable mitoxantrone solutions according to Claim 1, **characterized in that** the content of disodium edetate is preferably 0.004%.

4. Process for the production of stable mitoxantrone solutions according to any one of Claims 1 to 3, **characterized in that** sodium chloride, sodium acetate, acetic acid and sodium edetate are dissolved in low-oxygen water, mitoxantrone hydrochloride is introduced into one part of the solution, and the solutions are combined and adjusted to the final volume having a content of 1 to 5 mg of mitoxantrone hydrochloride/ml of solution and 0.001 to 0.15% of sodium edetate/ml of solution.

5. Process according to Claim 4, **characterized in that** 0.04% of sodium edetate is preferably added.

6. Process according to Claim 4, **characterized in that** 0.004% of disodium edetate is preferably added.

7. Use of the stable mitoxantrone solutions according to any one of Claims 1 to 3 for the production of a medicament for use in carcinomatous disorders.

8. Medicament for use in carcinomatous disorders, **characterized in that** it contains 1 to 5 mg of mitoxantrone hydrochloride/ml of solution, sodium edetate in an amount of 0.001 to 0.15%, based on mitoxantrone hydrochloride solution, and, as further constituents, sodium chloride, sodium acetate and acetic acid.

9. Medicament according to Claim 8, **characterized in that** it preferably contains 0.04% of sodium edetate.

10. Medicament according to Claim 8, **characterized in that** it preferably contains 0.004% of disodium edetate.

## Revendications

1. Solutions stables de mitoxantrone contenant 1 à 5 mg/ml de solution de chlorhydrate de mitoxantrone, 0,001 à 0,15% d'édétate de sodium et comme autres constituants du chlorure de sodium, de l'acétate de sodium et de l'acide acétique.

2. Solutions stables de mitoxantrone selon la revendication 1, **caractérisées en ce que** la teneur en édétate de sodium est de préférence 0,04% en poids.

3. Solutions d'injection stables de mitoxantrone selon la revendication 1, **caractérisées en ce que** la teneur en édétate de disodium est de préférence 0,004% en poids.

4. Procédé pour la préparation de solutions stables de mitoxantrone selon les revendications 1 et 2, **caractérisé en ce que** le chlorure de sodium, l'acétate de sodium, l'acide acétique et l'édétate de sodium sont dissous dans de l'eau pauvre en oxygène, le chlorhydrate de mitoxantrone est introduit dans une partie de la solution, les solutions sont réunies et réglées au volume final à une teneur de 1 à 5 mg de chlorhydrate de mitoxantrone/ml de solution et de 0,001 à 0,15% d'édétate de sodium/ml de solution.

5. Procédé selon la revendication 4, **caractérisé en ce qu'**on ajoute de préférence 0,04% d'édétate de sodium.

6. Procédé selon la revendication 4, **caractérisé en ce qu'**on ajoute de préférence 0,004% d'édétate de disodium.

7. Utilisation des solutions stables de mitoxantrone selon une de revendications 1 à 3 pour la préparation d'un médicament destiné à être utilisé pour des maladies cancéreuses.

8. Médicament destiné à être utilisé pour des maladies cancéreuses, **caractérisé en ce qu'**il contient 1 à 5 mg de chlorhydrate de mitoxantrone/ml de solution, de l'édétate de sodium en une quantité de 0,001 à 0,15% par rapport à la solution de chlorhydrate de mitoxantrone et comme autres constituants du chlorure de sodium, de l'acétate de sodium et de l'acide acétique.

9. Médicament selon la revendication 8, **caractérisé en ce qu'**il contient de préférence 0,04% d'édétate de sodium.

10. Médicament selon la revendication 8, **caractérisé en ce qu'**il contient de préférence 0,004% d'édétate de disodium.
